# EUROPEAN PATENT APPLICATION

(11) **EP 4 556 032 A1**
(43) Date of publication of application: **21.05.2025**
(21) Application number: 23839983.6
(22) Date of filing: 13.07.2023
(51) Int. Cl.: A61L 27/56, A61L 27/36

(54) **FIBROCARTILAGE-DERIVED BIOINK COMPOSITION, BONE GRAFT COMPOSITION CONTAINING SAME, AND MANUFACTURING METHOD THEREFOR**

(30) Priority: 13.07.2022 KR 20220086229
(71) Applicant: Ajou University Industry-Academic Cooperation Foundation, Suwon-si, Gyeonggi-do 16499 (KR)
(72) Inventor: PARK, Do Young, Suwon-si Gyeonggi-do 16516 (KR); YUN, Hee-Woong, Suwon-si Gyeonggi-do 16628 (KR); NOH, Sujin, Suwon-si Gyeonggi-do 16525 (KR)
(74) Representative: Mewburn Ellis LLP
(86) International application number: PCT/KR2023/009998
(87) International publication number: WO 2024/014892

(57) **Abstract**

The present invention relates to a fibrocartilage-derived bioink composition, a bone graft composition containing same, and a manufacturing method therefor. More specifically, it is identified in the present disclosure that the inner, mid, and outer compartments of the meniscal cartilage tissue based on anatomical location differ from each other in terms of physicochemical property, whereby use is made of the extracellular matrix derived from each compartmentalized tissue to provide 3D-printing bioink compositions and bone grafts, each having distinct physicochemical properties, which can be utilized with controlled physicochemical properties according to desired applications.

## Description

### Technical Field

The present disclosure relates to a histodifferentiation technique including a composite tissue-derived component, and more specifically, to a fibrocartilage-derived bioink composition, a bone graft composition including the same, and a manufacturing method thereof.

### Background Art

Meniscus tears are one of the most common orthopedic diseases, with a prevalence of approximately 20% in the middle-aged general population. The meniscus tears cause knee pain, function loss, and most importantly, acceleration of osteoarthritis in the knee joint. Much like articular cartilage defects, meniscus injury does not heal spontaneously, due to the limited blood supply especially in the inner two-thirds of the tissue. Current surgical treatments such as repair, partial meniscectomy, and allogeneic allograft transplantation fall short in fully restoring meniscus functions and prevent osteoarthritis. In turn, there is growing interest and clinical need to engineer the meniscus utilizing stem cells, biomaterials, and tissue engineering technologies.

Challenges regarding meniscus engineering can be owed to the complex morphology and functional demands of the meniscus. The meniscus resist stress from not only compressive, but also tensile, and shear forces within the knee joint, largely by the anisotropic and composite character of the meniscus tissue. Current meniscus engineering results, however, fall short in achieving biomimicry and functionality of the native meniscus, with challenges in reproducing a composite tissue that reflects innate properties of respective zones.

Use of decellularized tissue-derived extracellular matrix (ECM) based biomaterials may be a promising strategy for meniscus engineering. Decellularized ECM have shown to not only retain biochemical characteristics of the donor site tissue, but also regulate specific cellular mechanisms such as proliferation and differentiation, by providing tissue-specific factors that guide cell behavior towards donor site tissue. Regarding meniscus, various studies have been actively conducted on the biocompatibility and regenerability of the decellularized meniscus extracellular matrix (DMECM), but the exact composition of DMECM and characteristics of each are still unclear.

### Disclosure of the Invention

### Technical Goals

An object of the present disclosure is to provide a bioink for 3D printing capable of forming composite tissue with different physicochemical properties using a compartmentalized biotissue-derived material, and a manufacturing method thereof.

Another object of the present disclosure is to provide a bone graft in which the bioink is cross-linked to a bone scaffold, and a manufacturing method thereof.

### Technical Solutions

In order to achieve above objects, the present disclosure provides a bioink composition for 3D printing including an extracellular matrix derived from fibrocartilage composite tissue, wherein the fibrocartilage composite tissue is at least one selected from tissues compartmentalized into inner, mid, and outer meniscal tissues depending on anatomical positions, where the inner has cartilaginous properties, the outer has fibrous properties, and the mid has the property that the cartilage and fibrous properties are converted, such that physicochemical properties vary depending on the compartmentalized tissues.

The present disclosure provides a bone graft composition including a demineralized bone matrix scaffold wherein the bioink composition is included in the scaffold and cross-linked.

The present disclosure provides a method of manufacturing a bioink for 3D printing, including obtaining and pulverizing fibrocartilage composite tissue; decellularizing the pulverized tissue to obtain and pulverize extracellular matrix; and water-solubilizing the pulverized extracellular matrix by enzymatic treatment, wherein the fibrocartilage composite tissue is one or more selected from tissues compartmentalized into inner, mid, and outer meniscal tissues depending on anatomical positions, where the inner has cartilaginous properties, the outer has fibrous properties, and the mid has the property that the cartilage and fibrous properties are converted, such that physicochemical properties vary depending on the compartmentalized tissues.

The present disclosure provides a method of manufacturing a bone graft, including preparing a demineralized bone matrix scaffold; preparing a bioink including an extracellular matrix derived from fibrocartilage composite tissue according to the manufacturing method; and injecting the prepared bioink into the prepared demineralized bone matrix scaffold to carry out cross-linking.

In addition, the present disclosure provides a method of manufacturing an artificial tissue including 3D printing the bioink composition, and an artificial tissue prepared thereby.

### Advantageous Effects

By finiding that a bioink composition according to the present disclosure has different physicochemical properties in the inner, mid, and outer meniscal tissues compartmentalized depending on anatomical positions, respectively, it is possible to provide a bioink composition for 3D printing with different physicochemical properties using an extracellular matrix derived from each compartmentalized tissue.

A bone graft composition according to the present disclosure may provide a bone graft with physicochemical properties according to each compartment, by cross-linking the bioink with the demineralized bone matrix scaffold.

The bioink composition and bone graft according to the present disclosure have different characteristics for each compartment such as cartilage tissues, fibrous tissues, angiogenesis, and anti-angiogenic properties, and thus may be used by adjusting the physicochemical properties depending on the purpose.

### Brief Description of Drawings

FIG. 1(A) shows analyses and electron microscopic images of meniscal tissues by compartments, in which anatomical differences may be identified in the meniscal tissues by the inner, mid, and outer compartments, and FIG. 1(B) shows schematic diagrams of a manufacturing process of a zone-specific thermo-sensitive decellularized meniscus extracellular matrix (DMECM) bioink.
FIG. 2 shows results of analyzing biochemical characteristics of a zone-specific thermo-sensitive bioink (DMECM-bioink) prepared according to FIG. 1, presenting (A) a dsDNA content, (B) a collagen content, (C) sulfated glycosaminoglycan (sGAG), and (D) an elastin content, (E) a result of SDS-PAGE assay, and (F) a result of Western blot assay.
FIG. 3 shows analyses on rheological properties of a DMECM-bioink in accordance with temperature changes, presenting (A) changes in an elastic modulus with temperature changes, (B) morphological changes with temperature changes, and (C) the pre- and post-3D printing.
FIG. 4 shows identification of effects of a DMECM-bioink on cells, illustrating analyses on (A) cell migration ability (B) cell adhesion ability, (C) cell proliferation ability, and (D) cell affinity.
FIG. 5 shows identification of effects of a DMECM-bioink on differentiation of human synovial membrane-derived mesenchymal stem cells (hMSCs), illustrating changes in expressions of (A) type 1 collagen (COL1), (B) type 2 collagen (COL2), (C) aggrecan (ACAN), and (D) SOX9.
FIG. 6 shows assessments of the in vivo fibrocartilage differentiation ability of a DMECM-bioink, illustrating (A) the histological assessment of artificial tissues formed in vivo and (b) identification of a component content of the artificial tissue formed in vivo.
FIG. 7 shows assessments of in vitro angiogenesis induction ability of a DMECM-bioink on human umbilical vein endothelial cells, presenting analyses on (A-B) cell migration ability, (C-D) cell adhesion ability, (E-F) cell proliferation ability, and (G-I) tube formation capacity.
FIG. 8 shows schematic diagrams illustraeting a manufacturing process of a demineralized bone matrix (DBM) scaffold.
FIG. 9 shows schematic diagrams for a method of manufacturing an artificial meniscus scaffold mimicking the zone specificity of meniscus by 3D printing a thermo-sensitive DMECM-bioink onto a DBM scaffold.
FIG. 10(A) shows identification of the external appearance of a DBM scaffold by loading of zone-specific DMECM-bioink printing as well as an internal structure via SEM analysis, and FIGS. 10(B) to (D) show analyses on a component content of the DBM scaffold.
FIG. 11 shows analyses on physicochemical properties of a DBM scaffold loaded with a DMECM-bioink under cross-linking conditions, presenting (A-B) changes in a compressive modulus in accordance with cross-linkage, (C) a porosity of the scaffold in accordance with a concentration of the DMECM-bioink, (D) a pore size, (E) a swelling ratio, and (F) a cell seeding efficiency.
FIG. 12 shows physical characteristics of a DBM scaffold in accordance with a compact bone content, presenting (A) a surface structure, (B) a compressive modulus, and (C) a tensile modulus.
FIG. 13 shows results of analysis on a compressive modulus of a DBM scaffold printed with equal amounts of 5% Inner, Mid, and Outer DMECM-bioink, respectively.
FIG. 14 shows identification of effects of a zone-specific DMECM-DBM scaffold on cell behaviors, presenting analyses on (A) cell migration ability, (B) cell seeding rate, (C) cell proliferation ability, and (D) cell affinity.
FIG. 15 shows identification of effects of a zone-specific DMECM-DBM scaffold on differentiation of human synovial membrane-derived mesenchymal stem cells (hMSCs), presenting changes in expressions of (A) type 1 collagen (COL1), (B) type 2 collagen (COL2), (C) aggrecan (ACAN), and (D) SOX9.
FIG. 16 shows assessments of in vivo fibrocartilage differentiation ability of a DBM scaffold loaded with a zone-specific thermo-sensitive DMECM bioink, presenting (A) histological assessment of an artificial tissue formed in vivo and (b) identification of a component content of the artificial tissue formed in the vivo.
FIG. 17 shows schematic diagrams illustrating a manufacturing process of an artificial meniscus scaffold using a manufacturing technology for a DBM scaffold loaded with a zone-specific thermo-sensitive DMECM bioink.

### Best Mode for Carrying Out the Invention

Hereinafter, the present disclosure will be described in more detail.

The present inventor has completed the present disclosure by discovering that the inner, mid, and outer tissues compartmentalized by anatomical positions of the meniscus have significantly different physicochemical properties, respectively, and the compartmentalized tissue-derived extracellular matrix, when prepared as a bioink and a bone graft including the same, is usable by adjustment depending on the desired mechanical properties and biochemical properties.

The present disclosure provides a bioink composition for 3D printing including an extracellular matrix derived from fibrocartilage composite tissue.

As used herein, the term "3D (three-dimensional) printing" or "3D bioprinting" refers to a technology that enables formation of finely controllable 3D cell models or tissue constructs by designing a substrate in an anatomical morphology with a complexity similar to tissues.

As used herein, the term "bioink (bio-ink)" refers to those that are used in 3D bioprinting technology that involves the use of cells and biomaterials to create 3D artificial tissue structures, in that it should have physicochemical properties to maintain its microscopic 3D structure as well as features regarding biological safety at a level to be used along with living cells.

Preferably, the fibrocartilage composite tissue may be a meniscal tissue, and the meniscal tissue may be compartmentalized into the inner, mid, and outer according to anatomical positions.

According to an embodiment of the present disclosure, in the porcine meniscal tissue, the tissue with a width of 2-3 mm from the inside is defined as the inner, the tissue with a width of 4-6 mm from the outside as the outer, and a middle portion with a width of 7 to 9 mm between the inner and the outer as the mid, but are not limited thereto.

In addition to the anatomical position, the meniscal tissue may be, in addition to the anatomical positions, compartmentalized depending on the properties of the tissue, the degree of vascular infiltration, the difference in the microstructure, the difference in the content of biochemical components, and the difference by the distribution and type of cells, but is not limited thereto.

The meniscal tissue may have more fibrous characteristics than cartilaginous characteristics as going farther from the inner to the outer and have biochemical characteristics with significant differences.

More specifically, the inner has cartilaginous properties, the outer has fibrous properties, and the mid has the characteristic that the cartilage and fibrous properties are converted, such that the bioink composition including the compartmentalized tissue-derived extracellular matrix may have physicochemical properties that vary depending on the compartmentalized tissue.

According to an embodiment of the present disclosure, it was found that the bioink including the inner tissue-derived extracellular matrix of meniscal tissues showed relatively strong expression of type 2 collagen and aggrecan that are representative markers of the cartilage-derived extracellular matrix compared to the mid and outer, whereas the bioink including the mid and outer tissue-derived extracellular matrix showed stronger expression of type 1 collagen, a representative marker of fibrous tissue, than that of the inner.

In addition, according to another embodiment of the present disclosure, the inner tissue of the meniscal tissue has the highest content of sulfated glycosaminoglycan (sGAG), the mid tissue has a high total collagen content, and the outer tissue has the highest elastin content.

In other words, since the physicochemical properties is controllable by selecting the compartmentalized tissue differently for the purpose, the bioink composition according to the present disclosure may have zone-specificity, including the extracellular matrix derived from tissues selected from the inner tissue, the mid tissue, the outer tissue, or a combination thereof.

The bioink composition according to the present disclosure may promote the migration, adhesion, and proliferation of fibrocartilage cells, and differentiate into fibrocartilage composite tissue in vitro or in vivo to induce the formation of artificial tissues.

Moreover, the bioink composition according to the present disclosure may have angiogenesis-inducing or anti-angiogenic properties.

According to an embodiment of the present disclosure, it was found that the inner or mid tissue-derived bioink composition exhibits anti-angiogenic properties such as inhibition of cell migration and cell adhesion of vascular endothelial cells, while the outer tissue-derived bioink composition has a chemotaxis for vascular endothelial cells, significantly promotes cell adhesion, and has pro-angiogenic properties as cell proliferation takes place in a net-like structure similar to a capillary network.

The supply of nutrients through angiogenesis and the induction of stem cell migration are very important for meniscus reconstruction. In particular, the synovial membrane adj acent to the outer portion of the meniscus may be a rich source of blood, nutrients and stem cells, and the induction of angiogenesis from the adjacent area to the inside of the meniscal tissue may play an important role in the regeneration of the entire meniscal tissue, such that the bioink composition according to the present disclosure may satisfy these characteristics and thus is desirable.

In other words, the bioink composition according to the present disclosure has properties of inducing fibrous tissues, cartilage tissues, and angiogenesis or anti-angiogenic properties by compartments, and thus may be useful for regeneration of fibrocartilage composite tissue by using these zone-specificity.

In addition, the bioink composition according to the present disclosure may exist in a liquid form at room temperature and then undergo gelation at a temperature greater or equal to 37°C, preferably at a temperature from 37°C to 45°C, thereby having a thermos-sensitivity to retain a certain morphology.

As described above, since the bioink for 3D printing should have physicochemical properties such as printability to be extruded from a 3D printer and rheological properties to maintain 3D structure even after printing, have cell affinity and biocompatibility because it needs to function as a scaffold for cell growth and differentiation, and also possess properties to regulate the growth and differentiation of cells in environment for in vitro culture or in vivo transplantation, the bioink composition according to the present disclosure may satisfy these features and thus is desirable.

The present disclosure provides a bone graft composition including a demineralized bone matrix scaffold.

As used herein, the term "demineralized bone matrix (DBM)" refers to an allogeneic bone graft that has been demineralized, and when grafted alone, it is difficult to maintain its morphology, with insufficient durability and compressive modulus.

The demineralized bone matrix scaffold may include spongy bone, compact bone, or a combination thereof, and preferably may include both spongy bone and compact bone, but is not limited thereto.

The demineralized bone matrix scaffold may be in the form of compact bone and spongy bone combined in a layered structure, and the compact bone and spongy bone may be combined in an average thickness ratio of 1:(2 to 5), but is not limited thereto.

According to an embodiment of the present disclosure, it may be determined that the compressive modulus and tensile modulus of the demineralized bone matrix scaffold containing both spongy bone and compact bone are significantly increased than the demineralized bone matrix scaffold containing only the spongy bone.

Preferably, the bone graft composition may be those in which the bioink composition is included in the demineralized bone matrix scaffold.

The bioink composition may have a concentration of 0.1 to 10 weight-volume (w/v) percent, preferably a concentration of 2 to 5 weight-volume (w/v) percent, but is not limited thereto.

The demineralized bone matrix scaffold may be formed in a porous structure, and the bioink composition may be contained in the porous structure of the scaffold and may be those cross-linked in a hydrogel state in accordance with temperature changes.

Preferably, the bioink composition may be 3D printed on the demineralized bone matrix scaffold.

Since the bioink composition has different biochemical properties by compartment of the meniscal tissue, with the mechanical properties controllable, the compartmentalized tissue may be selectively adjusted depending on the purpose to be contained in the cross-linked in the demineralized bone matrix scaffold.

According to an embodiment of the present disclosure, the inner tissue-derived bioink composition has superior compressive modulus compared to other compartments, and thus may be selectively used in tissue regeneration where physical compressive modulus must be biomechanically maintained.

In addition, the bone graft composition may be utilized for 3D bioprinting, and the physicochemical properties may be further improved through physical or chemical cross-linking.

The present disclosure provides a method of manufacturing a bioink for 3D printing, including an extracellular matrix derived from fibrocartilage composite tissue.

The method of manufacturing a bioink according to the present disclosure includes obtaining and pulverizing fibrocartilage composite tissue; decellularizing the pulverized tissue to obtain and pulverize extracellular matrix; and water-solubilizing the pulverized extracellular matrix by enzymatic treatment.

The obtaining and performing of the fibrocartilage composite tissue may be performed by obtaining the meniscal tissue, preferably it may be performed by pulverizing after obtaining the meniscal tissues that are compartmentalized into the inner, mid, and outer depending on the anatomical position.

In addition to the anatomical position, the compartmentation of tissues may be carried out according to the physical properties of the tissue, the degree of vascular infiltration, the difference in microstructure, the difference in the content of biochemical components, and the difference by the distribution and type of cells, but is not limited thereto.

The pulverization may be carried out by washing each tissue obtained or a tissue mixture thereof with distilled water more than three times, and then performing lyophilization and cryo-crushing.

In the decellularizing of the pulverized tissue to obtain and pulverize the extracellular matrix, decellularization, a process of removing the cells and genetic materials present in the tissue to obtain a pure extracellular matrix, may be performed by first putting the pulverized tissue into a hypotonic solution for 3 to 5 hours of treatment at room temperature, and then putting in a surfactant solution for 1 to 3 hours of treatment. After washing with distilled water to remove the surfactant, decellularization may be completed by treatment with a DNase solution for 10 to 15 hours to remove the genetic materials present in the extracellular matrix, followed by washing.

The water-solubilizing of the pulverized extracellular matrix by enzymatic treatment may be performed by water-solubilizing the pulverized extracellular matrix through enzymatic treatment such as pepsin in an acidic solution, and, after re-neutralization, diluting with a buffer solution to adjust the concentration of physiological ions. The water-solubilized extracellular matrix may be prepared in an amount of 0.1 to 10 % (w/v), preferably in an amount of 2 to 5 % (w/v), but is not limited thereto.

The bioink prepared according to the manufacturing method may exist in a liquid form at room temperature and undergo gelation at temperatures greater or equal to 37°C, thereby having a thermo-sensitivity to retain specific morphology.

The present disclosure provides a method of manufacturing a bone graft, including a demineralized bone matrix scaffold in which the bioink composition is contained.

The method of manufacturing a bone graft according to the present disclosure may include preparing a demineralized bone matrix scaffold; preparing a bioink including an extracellular matrix derived from fibrocartilage composite tissue; and injecting the prepared bioink into the prepared demineralized bone matrix scaffold to carry out cross-linking.

The preparing of the demineralized bone matrix scaffold may include obtaining an osteo-cartilage composite tissue and then demineralizing the composite tissue; and removing cartilage tissues from the demineralized composite tissue.

The demineralizing may be carried out by treating an acidic solution to remove minerals or calcium.

After removing the cartilage tissue, the demineralizing may further include performing washing for removal of cells and foreign substances.

The demineralized bone matrix scaffold prepared according to the method may include spongy bone, compact bone, or a combination thereof, and preferably may include both spongy bone and compact bone, but is not limited thereto.

The preparing of the bioink may be replaced by the foregoing method of manufacturing a bioink for 3D printing.

The carrying out of the cross-linking by injecting the prepared bioink into the prepared demineralized bone matrix scaffold may be performed, after the bioink is injected into a porous structure in the demineralized bone matrix scaffold, by cross-linking in accordance with the temperature changes, preferably at a temperature of 37°C or higher in a hydrogel state. Alternatively, it may be performed by 3D printing the bioink onto the prepared demineralized bone matrix scaffold.

The method of manufacturing a bone graft may further include additionally cross-linking the cross-linked bioink to a porous structure in the demineralized bone matrix scaffold using one or more physical or chemical cross-linking methods.

The physical cross-linking method may be performed by irradiating gamma rays, and the chemical cross-linking method may be performed by using a cross-linking agent such as glutaraldehyde.

According to an embodiment of the present disclosure, as the concentration of the cross-linking agent increases or the irradiated dose of gamma rays increases, it may be seen that the compressive modulus of the demineralized bone matrix scaffold to which the bioink is cross-linked increases significantly.

Moreover, as the concentration of bioink injected into the demineralized bone matrix scaffold increases, it was found that the compressive modulus of the scaffold significantly increases, while the porosity and pore size decrease.

In other words, it is possible to control physicochemical properties such as mechanical properties, porosity, and swelling depending on the purpose by the manufacturing method according to the present disclosure and manufacture a bone graft in which the cell seeding rate is also adjustable according to the control of these properties.

In addition, the present disclosure provides a method of manufacturing an artificial tissue by 3D printing a bioink composition for 3D printing including the extracellular matrix derived from the fibrocartilage composite tissue.

More specifically, the 3D printing may be performed at a rate of 1 to 2 mm/sec at an air pressure of less than 80 kPa and a temperature of 37°C, using a nozzle with an average diameter of 300 to 600 µm.

The present disclosure provides an artificial tissue obtained by 3D printing the bioink composition.

The artificial tissue may have the characteristics of the fibrocartilage composite tissue.

The artificial tissue may be an artificial meniscal tissue, but is not limited thereto. Modes for Carrying Out the Invention

Hereinafter, the present disclosure will be described in detail through examples to help understanding of the present disclosure. However, examples below are merely intended to illustrate the present disclosure, and the scope of the present disclosure is not limited to the following examples. Examples of the present disclosure are provided to more completely explain the present disclosure to those skilled in the art.

### <Example 1> Preparation of a zone-specific thermo-sensitive decellularized meniscus extracellular matrix (DMECM) bioink

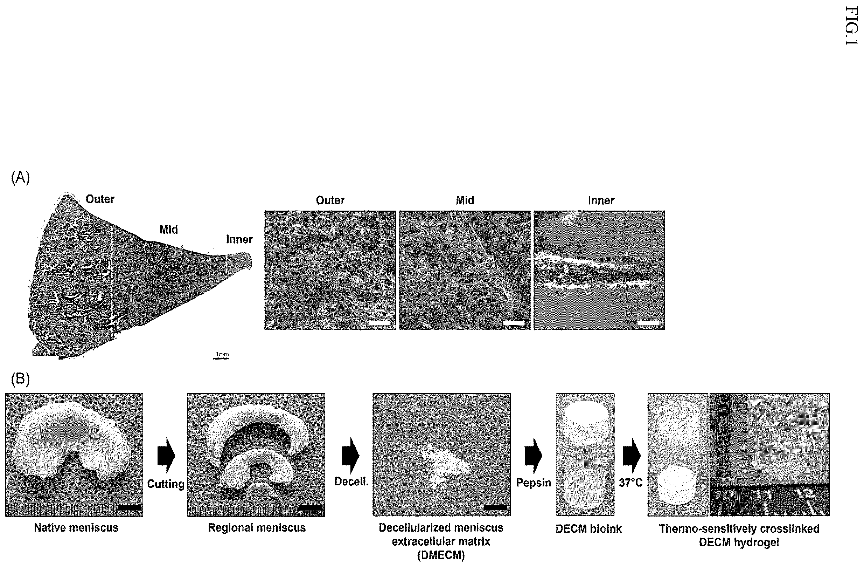

Porcine meniscal tissue is a fibro-cartilage composite connective tissue with different tissue characteristics in the inner, mid, and outer (FIG. 1A).

Referring to FIG. 1(B), to prepare a zone-specific thermo-sensitive decellularized meniscus extracellular matrix (DMECM) bioink, meniscal tissues were first harvested from pig knees using surgical blades and saws. Subsequently, based on the anatomical features of the meniscus, the tissue with a width of 2-3 mm from the inner meniscus was defined as the inner zone, the tissue with a width of 4-6 mm from the outer as the outer zone, and the mid with a width of 7-9 mm between the inner and outer zones as the mid zone.

Zone-specific meniscal tissues were washed three times with distilled water and then obtained in the powder form through lyophilization and freezer milling. The obtained meniscal tissue powder was treated through addition of a hypotonic solution for 4 hours at room temperature, and then treated with a surfactant solution including sodium dodecyl sulfate (SDS) for 2 hours to carry out decellularization. Afterwards, washing was performed 6 times with distilled water to remove SDS. Finally, in order to remove the genetic materials that are present in the tissue extracellular matrix powder, a solution containing DNase was treated for 12 hours. Afterwards, additional washing was followed 6 times with distilled water to complete the decellularization process. Subsequently, after water-solubilizing the extracellular matrix in the powder form through pepsin treatment in 0.1M HCl solution, the pH was neutralized with NaOH, and the concentration of physiological ions was adjusted by properly diluting with highly concentrated phosphate buffered saline (PBS).

The finally prepared decellularized meniscus extracellular matrix (DMECM)-bioink showed the characteristics of a thermo-sensitive hydrogel that exhibits the properties of a liquid at room temperature (below 25°C) and becomes gelated at temperatures from 37°C to 45°C as shown in FIG. 1(B).

### <Example 2> Analysis on biochemical characteristics of the zone-specific thermo-sensitive DMECM bioink

### 2-1. Analysis on the content of dsDNA, collagen, sGAG, and elastin in the zone-specific DMECM-bioink

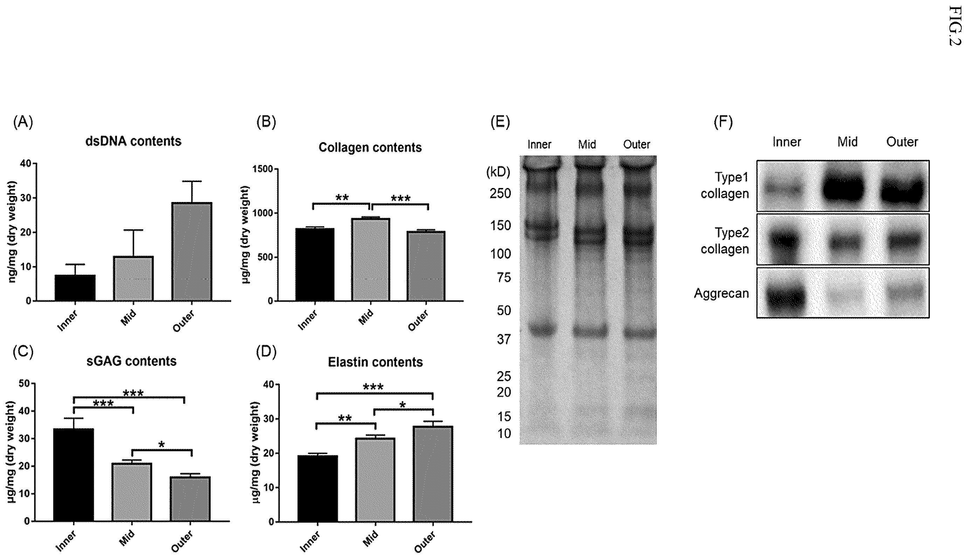

The dsDNA content of the Inner, Mid, and Outer DMECM-bioink was analyzed using a PicoGreen assay. As a result, as shown in FIG. 2(A), all bioinks showed dsDNA levels less than or equal to 50 ng per mg. Thereby, it may be determined that the three tissues above have been successfully decellularized.

FIGS. 2(B) to 2(D) are quantitative results obtained via biochemical analysis on the content of total collagen, sulfated glycosaminoglycan (sGAG), and elastin of the zone-specific DMECM-bioink, and, referring to the result, the collagen content was shown to be significantly high in the Mid group compared to the Inner and Outer groups (Mid: 935.02 ± 21.3 µg/mg, Inner: 821.01 ± 23.8 µg/mg, Outer: 786.05 ± 26 µg/mg). In case of sGAG content, the Inner group had the highest sGAG content compared to the other groups, and the quantitative value of sGAG decreased in the order of the Mid and Outer DMECM-bioink (Inner: 33.4 ± 4 µg/mg, Mid: 20.9 ± 1.3 µg/mg, Outer: 15.9 ± 1.2 µg/mg). In case of elastin, it was identified that the content increased from the inner tissue to the outer tissue (Inner: 19.1 ± 0.8 µg/mg, Mid: 24.2 ± 1.01 µg/mg, Outer: 27.7 ± 1.5 µg/mg).

### 2-2. Protein analysis in the zone-specific DMECM-bioink

SDS-PAGE analysis was performed to analyze the differences in the protein cargo profile of the DMECM-bioink by zone. As a result, as shown in FIG. 2(E), the position of the collagen chain band in the inner group was found to be higher than that in the other groups.

Subsequently, as a result of comparative analysis on the relative expression patterns of type 1 collagen, type 2 collagen, and aggrecan included in the zone-specific DMECM-bioink through western blot, it may be seen, as shown in FIG. 2(F), that the Inner DMECM-bioink caused relatively stronger expression of type 2 collagen and aggrecan, which are representative markers of cartilage-derived extracellular matrix, than that of the Mid and Outer DMECM-bioink. On the other hand, in the case of the Mid and Outer DMECM-bioink, the expression of type 1 collagen, a representative marker of fibrous tissues, was shown to be stronger than that of the Inner DMECM-bioink.

Thereby, it is indicated that DMECM-bioinks in the Inner, Mid, and Outer groups, separated according to the anatomical position of the meniscus, show significant differences in biochemical characteristics.

### <Example 3> Analysis of proteomic characteristics of the zone-specific thermo-sensitive DMECM bioink

Table 1 below shows a result of the proteomics analysis of the zone-specific thermo-sensitive DMECM bioinks, represented in numbers for the relative expression levels of proteins present in the Inner, Mid, and Outer DMECM-bioinks, respectively.

**TABLE 1**

| **Protein name** | **Mapped ID** | **Molecular Weight** | **In** | **Mid** | **Out** |
|---|---|---|---|---|---|
| **Aggrecan core protein** | ACAN | 238 kDa | 630.16 | 155.72 | 84.437 |
| **Aggrecan core protein (Fragments)** | ACAN | 59 kDa | 319.87 | 0 | 0 |
| **Annexin** | ANXA1 | 39 kDa | 7.9494 | 8.9307 | 20.06 |
| **Biglycan** | BGN | 42 kDa | 210.93 | 326.56 | 309.08 |
| **Chitinase-3-like protein 1** | CHI3L1 | 43 kDa | 2.0704 | 0 | 0 |
| **Cartilage Intermediate layer protein 1** | CILP | 133 kDa | 78.296 | 44.464 | 7.9119 |
| **Cluster of Tenascin** | TNC | 191 kDa | 608.26 | 570.14 | 457.5 |
| **Cluster of TGF-beta-induced protein ig-h3** | TGFBI | 74 kDa | 1,124.40 | 577.4 | 428.83 |
| **Collagen alpha-1(III) chain preproprotein** | COL3A1 | 139 kDa | 176.82 | 304.61 | 307.69 |
| **Collagen alpha-2(V) chain** | COL5A2 | 145 kDa | 15.5 | 3.7802 | 0.96567 |
| **Collagen type XXI alpha 1** | COL21A1 | 93 kDa | 5.6035 | 9.3559 | 14.266 |
| **Fibrillar collagen NC1 domain-containing protein** | COL1A2 | 129 kDa | 511.31 | 936.84 | 906.57 |
| **Fibrillar collagen NC1 domain-containing protein** | COL2A1 | 130 kDa | 18.605 | 2.3153 | 0 |
| **Fibrillin-1** | FBN1 | 313 kDa | 14.132 | 4.7253 | 7.7253 |
| **Hyaluronan and proteoglycan link protein 1** | HAPLN1 | 40 kDa | 102.91 | 40.305 | 14.825 |
| **Lumican** | LUM | 39 kDa | 26.251 | 9.6631 | 7.2261 |
| **Protein disulfide-isomarase** | | 56 kDa | 0 | 0 | 13.487 |
| **Tenascin** | TNC | 191 kDa | 601.78 | 567.3 | 456.54 |
| **Tenascin XB** | TNXB | 447 kDa | 127.86 | 218.33 | 444.38 |
| **TGF-beta-induced protein ig-h3** | TGFBI | 74 kDa | 1,009.70 | 490.57 | 360.18 |
| **Type VI collagen alpha-1 chain** | COL6A1 | 10 kDa | 128.41 | 140.05 | 160.41 |
| **Versican** | VCAN | 262 kDa | 1.0352 | 3.4729 | 12.708 |
| **Vimentin** | VIM | 54 kDa | 17.874 | 118.44 | 199.44 |
| **Vitrin isoform 6** | VIT | 62 kDa | 6.2111 | 0 | 0 |
| **Vitronectin** | VTN | 28 kDa | 7.2463 | 5.8829 | 0 |

Referring to Table 1, it can be identified, in the protein analysis through proteomics, that the expression of type 2 collagen (COL2A1), aggrecan (ACAN), tenascin (TNC), and cartilage intermediate layer protein 1 (CLIP) representing the extracellular matrix (ECM) of cartilage was predominant in the Inner DMECM-bioink compared to the Mid and Outer DMECM-bioink.

On the other hand, expression of proteins such as type 1 collagen (COL1A2), vimentin (VIM), annexin A2 (ANXA2), and tubulin alpha chain (TUBA1C) was predominant in the Mid and Outer DMECM-bioink compared to the Inner DMECM-bioink.

### <Example 4> 3D printing of the thermo-sensitive DMECM bioink

In order to investigate the rheological properties of the thermo-sensitive DMECM-bioink by temperature changes, the shear rate was maintained at 1 s⁻¹, and the gelation rheology was measured by varying the temperature of the analytical instrument from 4°C to 45°C.

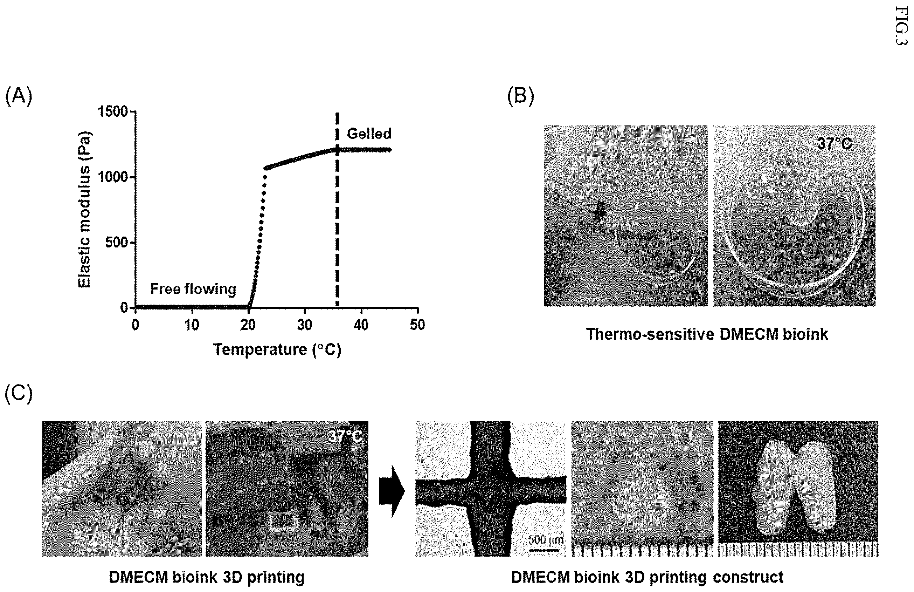

As a result, as shown in FIG. 3, it was found that the elastic modulus of the DMECM-bioink increased remarkably at temperatures of 20°C or higher following thermal gelation of collagen-rich DMECM-bioink (FIG. 3A). Since the DMECM-bioink has thermo-sensitivity, it was found that administration may be practiced freely in a desired amount at room temperature, with a specific morphology retained at 37°C (FIG. 3B).

In order to prepare a contruct in a desired shape by using the thermo-sensitive DMECM-bioink in the 3D printer, the printing conditions were set as follows. In consideration of the thermo-sensitivity, viscosity, and printability of the DMECM-bioink, the nozzle size was set to be no more than 300 µm to 600 µm, with an air pressure of less than 80 Kpa, a printing rate of 1 mm/sec, and a temperature of a lower plate of the 3D printer at 37°C.

As a result, as shown on the right of FIG. 3(C), it was determined that it is possible to produce not only a fine cross-shaped structure with a size of 500 µm but also a large structure with a size of 1 cm or greater and a complex cartilage-shaped structure.

### <Example 5> Cellular affinity of the zone-specific thermo-sensitive DMECM bioink

To assess the difference in the effect of the zone-specific thermo-sensitive DMECM-bioink on cell behaviors, analysis was conducted on cell proliferation and viable migration adhesion.

### 5-1. Analysis on the fibro-cartilage cell migration ability of the zone-specific thermo-sensitive DMECM-bioink

To identify that the zone-specific thermo-sensitive DMECM-bioink has biochemical chemotaxis and is capable of promoting migration of the meniscus-derived cells (meniscal cells), Boyden chamber assay was performed.

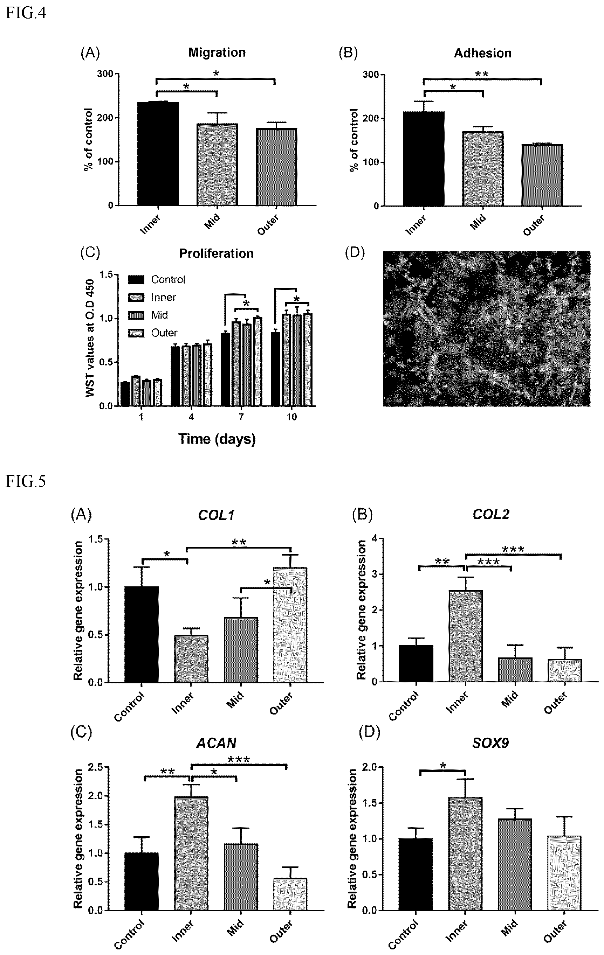

As a result, as shown in FIG. 4(A), it was found that all DMECM-bioinks significantly promoted migration of meniscal cells compared to the control group (cell culture: 100%). Particularly, the Inner DMECM-bioink showed the most significant chemotaxis for meniscal cells among all groups, followed by the Mid and Outer DMECM-bioinks.

### 5-2. Analysis on fibro-cartilage cell adhesion of the zone-specific thermo-sensitive DMECM-bioink

To assess cell adhesion, 5% DMECM-bioink was dispensed into a culture dish and incubated at 37°C for 30 minutes to coat the surface of the culture dish. Subsequently, 2 hours after seeding the meniscal cells, the culture dish was washed twice with PBS, and the cells adhered to the surface of the bioink were stained with Calcein-AM to measure absorbance.

As a result, as shown in FIG. 4(B), all DMECM-bioink treated groups significantly promoted cell adhesion compared to the control group (culture dish: 100%), and in particular, it was found that the Inner DMECM-bioink group showed the highest cell adhesion rate.

### 5-3. Analysis of fibro-cartilage cell proliferation ability of the zone-specific thermo-sensitive DMECM-bioink

In order to identify the cell proliferation ability of the zone-specific DMECM-bioink on meniscal cells, 1×10⁴ meniscal cells per 200 µl of 5% DMECM-bioink were suspended and seeded in a 96-well plate with a cell culture at 37°C, and proliferation of cells on days 1, 4, 7, and 10 was analyzed by WST assay.

As a result, as shown in FIG. 4(C), it was found that cell proliferation increased in all groups of DMECM-bioink treated groups compared to the control group with no treatment.

Moreover, to analyze the long-term cell affinity of the zone-specific DMECM-bioink on meniscal cells, LIVE & DEAD assay was performed 4 weeks after cell culture.

As a result, it was found that the cells survived (Green) on the inside and the surface of the bioink without the death (Red) of meniscal cells as shown in FIG. 4(D).

### <Example 6> In vitro fibrocartilage differentiation induction properties of the zone-specific thermo-sensitive DMECM bioink

The RT-PCR assay was performed to assess how zone-specific DMECM-bioink treatment affects the differentiation of human synovial membrane-derived mesenchymal stem cells (hMSCs).

As a result, as shown in FIG. 5, the expression of type 2 collagen (COL2), aggrecan (ACAN), and SOX9, which are known to be representative markers of cartilage tissues, was shown to be relatively higher in the Inner DMECM-bioink treated group compared to the Mid and Outer DMECM-bioink groups. On the other hand, the expression of type 1 collagen (COL1) was found to be lowest in the Inner DMECM-bioink group and becomes higher in the order of the Mid to the Outer DMECM-bioink.

### <Example 7> Assessment of in vivo fibrocartilage differentiation ability of the zone-specific thermo-sensitive DMECM bioink

### 7-1. Histological assessment of the artificial tissues formed subcutaneously in nude mice

In order to assess the in vivo artificial tissue formation capacity, hMSC and 2% zone-specific DMECM-bioink were suspended and injected subcutaneously in the nude mice at 1×10⁶ /100 µl each. Four weeks after subcutaneous injection, visual observation and histological assessment were conducted to assess the degree of tissue formation and differentiation by sacrificing the nude mice.

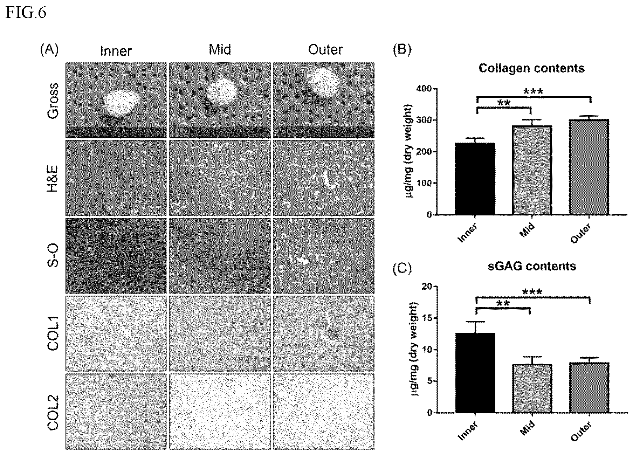

As a result, as shown in FIG. 6(A), it was found that a single homogeneous artificial tissue was formed at a site of injection of cell-zone-specific DMECM-bioink. In addition, as a result of H&E staining, it was found that the homogeneous cytoplasm was formed, and the cell distribution was also uniform. Moreover, it was found that staining patterns of safranin-O staining and expression patterns of collagen type, similar to the anatomical position of fibro-cartilage where the extracellular matrix originates, also appeared in artificial tissues.

### 7-2. Identification of the component content of artificial tissues formed subcutaneously in the nude mice

Analysis of collagen and sGAG was performed to determine the component content of the artificial tissues formed in vivo following injection of hMSC and the zone-specific DMECM-bioink.

As a result, as shown in FIG. 6(B), collagen content was measured highest in the Outer DMECM-bioink group and lowest in the Inner DMECM-bioink group (Inner: 225.8 ± 17.4 µg/mg, Mid: 280.8 ± 20.6 µg/mg, Outer: 300.8 ± 12.2 µg/mg).

On the other hand, sGAG showed the highest values in the Inner DMECM-bioink group, and there was no significant difference in the Mid and Outer DMECM-bioink (Inner: 12.5 ± 1.94 µg/mg, Mid: 7.6 ± 1.2 µg/mg, Outer: 7.8 ± 0.9 µg/mg).

<Example 8> Assessment of induction of in vivo angiogenesis by the zone-specific thermo-sensitive DMECM bioink

To assess the difference of the zone-specific DMECM-bioink on the induction of angiogenesis, analysis was conducted on migration, adhesion, proliferation and angiogenesis of human umbilical vein endothelial cells (HUVECs).

8-1. Analysis of migration ability of human umbilical vein endothelial cells by the zone-specific DMECM-bioink

To determine whether the zone-specific DMECM-bioink has biochemical chemotaxis and is capable of promoting or inhibiting the migration of human umbilical vein endothelial cells (HUVECs), Boyden chamber assay was performed.

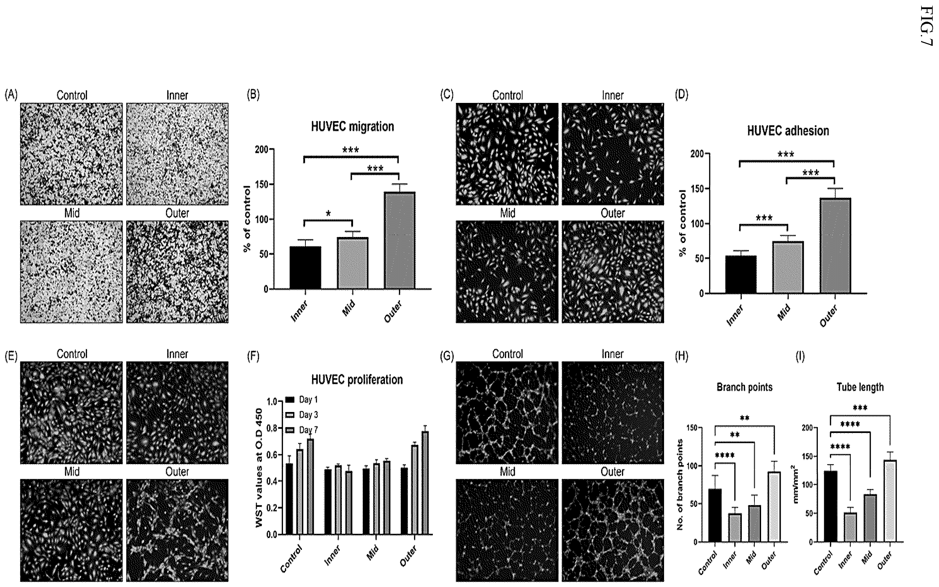

As a result, as shown in FIGS. 7(A) and (B), it may be found that the Inner and Mid DMECM-bioink significantly inhibits the migration of the human umbilical vein endothelial cells compared to the control group (cell culture containing 10% FBS: 100%) (Compared to the control group, Inner: approximately 39.2% reduction, Mid: approximately 26% reduction).

On the other hand, the Outer DMECM-bioink showed the most significant chemotaxis for human umbilical vein endothelial cells among all groups (Compared to the control group, Outer: 39.3% increase).

8-2. Analysis of adhesion of human umbilical vein endothelial cells by the zone-specific DMECM-bioink

To assess cell adhesion, 5% DMECM-bioink was dispensed into a culture dish and incubated at 37°C for 30 minutes to coat the surface of the culture dish. Subsequently, 2 hours after seeding the human umbilical vein endothelial cells, the culture dish was washed twice with PBS, and the cells adhered to the surface of the bioink were stained with Calcein-AM to measure absorbance.

As a result, as shown in FIGS. 7(C) and (D), the Inner and Mid DMECM-bioink treated groups significantly inhibited cell adhesion compared to the control group (culture dish: 100%), and the Outer DMECM-bioink significantly promoted the adhesion of human umbilical vein endothelial cells (Inner: 53.8 ± 7.02%, Mid: 74.8 ± 7.9%, Outer: 137.3 ± 12.9%).

### 8-3. Analysis of proliferation ability of human umbilical vein endothelial cells by the zone-specific thermo-sensitive DMECM-bioink

In order to identify the cell proliferation ability of the zone-specific DMECM-bioink on human umbilical vein endothelial cells, 200µl of 5% DMECM-bioink was coated on the 96-well plate followed by incubation at 37°C, and 1×10⁴ human umbilical vein endothelial cells per well were seeded with cell culture to analyze the proliferation of cells on days 1, 3 and 7 via WST assay, followed by staining of the cell morphology with Calcein-AM on day 7.

As a result, as shown in FIG. 7(E), it was observed that the control group with no treatment and the Inner and Mid DMECM-bioink treated groups were cultured in a round or oval form adhered to the cell culture dish and each surface of the DMECM-bioink. On the other hand, it was observed that the human umbilical vein endothelial cell morphology of the Outer DMECM-bioink treated group was relatively elongated compared to the other groups and arranged in a net-like structure similar to a capillary network.

In the cell proliferation analysis via WST assay, as shown in FIG. 7(F), the proliferation of human umbilical vein endothelial cells was observed significantly in the control group and the Outer DMECM-bioink treated group according to the culture period. On the other hand, in the Inner and Mid DMECM-bioink treated groups, the WST absorbance for early human umbilical vein endothelial cells was maintained over the culture period, with no significant changes observed in cell proliferation.

### 8-4. In vitro angiogenesis inducibility of human umbilical vein endothelial cells by the zone-specific thermo-sensitive DMECM-bioink

To identify the in vitro angiogenesis inducibility of the zone-specific DMECM-bioink on human umbilical vein endothelial cells, and Matrigel and 5% DMECM-bioink were suspended in a ratio of 9:1 and then coated by 100µl on a 96-well plate, followed by incubation at 37°C. Afterwards, 4×10⁴ human umbilical vein endothelial cells per well were seeded with cell culture, and, after 24 hours, the tube formation of human umbilical vein endothelial cells was stained with Calcein-AM and observed under a microscope. For the neovascular networks generated in vitro, the total length and number of branch points of the created vascular tubes were quantitatively analyzed using image analyzing software (ImageJ) to assess the degree of promotion or inhibition of neovascularization.

As a result, it was found that a tubular structure similar to a structure of the vascular network was formed in the control group treated with Matrigel alone, as shown in FIGS. 7(G) to (I). On the other hand, in the Outer DMECM-bioink treated group, it was found that the entire tube length and the number of branch points increased significantly compared to the control group. On the other hand, in the case of the Inner and Mid DMECM-bioink, it was observed that the structure of the tube was poorly formed compared to the control group, and the branch point and tube length were significantly reduced compared to the control group.

Thus, the Inner and Mid DMECM-bioink showed anti-angiogenic properties, while the Outer DMECM-bioink showed pro-angiogenic properties, revealing controllability of the DMECM-bioink for enhancing or inhibiting angiogenesis at the desired graft site according to the compartment of the native tissue.

### <Example 9> Preparation of a demineralized bone matrix (DBM) scaffold

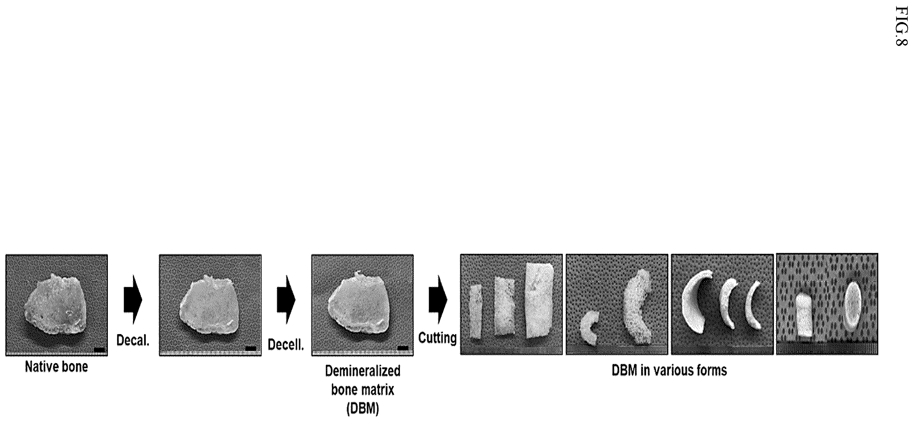

Demineralized bone matrix (DBM) tissues may be obtained from animal tissue, including humans, preferably from osteo-cartilage tissues obtained from cadavers, patients undergoing artificial joint surgery, or pigs. The obtained osteo-cartilage tissue was treated with a 5% nitric acid solution at 4°C for 48 hours for removal of mineral and calcium. The nitric acid solution was replaced every 24 hours. After mineral and calcium removal, the cartilage tissue section was removed from the osteo-cartilage tissue using a surgical blade, and the separated bone tissue was washed with a 15% triton-x 100 solution at 4°C for 72 hours for cell elimination and foreign substance removal. Triton-x 100 solution was replaced every 24 hours. In order to remove the residual Triton-x 100 after washing, stirring was performed twice with third water for 24 hours in total of 48 hours (FIG. 8).

DBM with demineralization and decellularization completed may be cut and shaped to fit the desired morphology and size. In the case of fabricating a DBM scaffold with a 3D curved surface, it is possible to immobilize the wet DBM onto the curved mold, followed by freeze-drying to mold in the desired shape.

In addition, the bone matrix has a structure in which spongy bone and compact bone are combined, where the spongy bone ensures easiness in shaping the scaffold due to high porosity, excellent tensile modulus, and elasticity, and the compact bone has favorable physical strength owing to low porosity and hardness. Depending on the intended use of the scaffold, only the spongy bone may be selectively used, or both the spongy bone and compact bone may be used.

### <Example 10> Preparation of a demineralized bone matrix (DBM) scaffold loaded with the zone-specific thermo-sensitive DMECM bioink

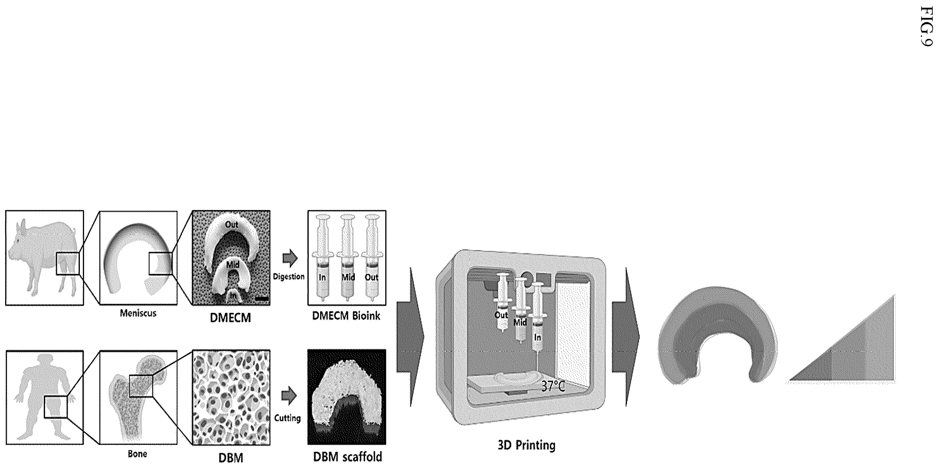

Referring to FIG. 9, DMECM-bioink 3D printed on the surface of the DBM scaffold is soaked into the porous structure of the DBM scaffold and then cross-linked in a hydrogel state by means of the lower thermostat that is set to 37°C. The concentration of the DMECM-bioink used in the bioprinting may be 0.1 to 10% (w/v), or more preferably 2 to 5%, but is not limited thereto. The DBM used in the bioprinting may be a spongy bone including a spongy bone or a compact bone layer. The DMECM-bioink cross-linked to the porous structure in the DBM scaffold at temperatures of 37°C or higher may improve the physicochemical properties of the scaffold through additional physical or chemical cross-linking methods.

### <Example 11> Microstructure and biochemical properties of the DBM scaffold loaded with the zone-specific thermo-sensitive DMECM bioink

In order to analyze the structure of the DBM scaffold according to the loading of the zone-specific DMECM-bioink printing, the DBM-DMECM scaffold was fabricated under conditions to print with each zone-specific DMECM-bioink prepared at 5 % (w/v) on a cylindrical DBM scaffold fabricated in a diameter of 5 mm and a height of 2 mm.

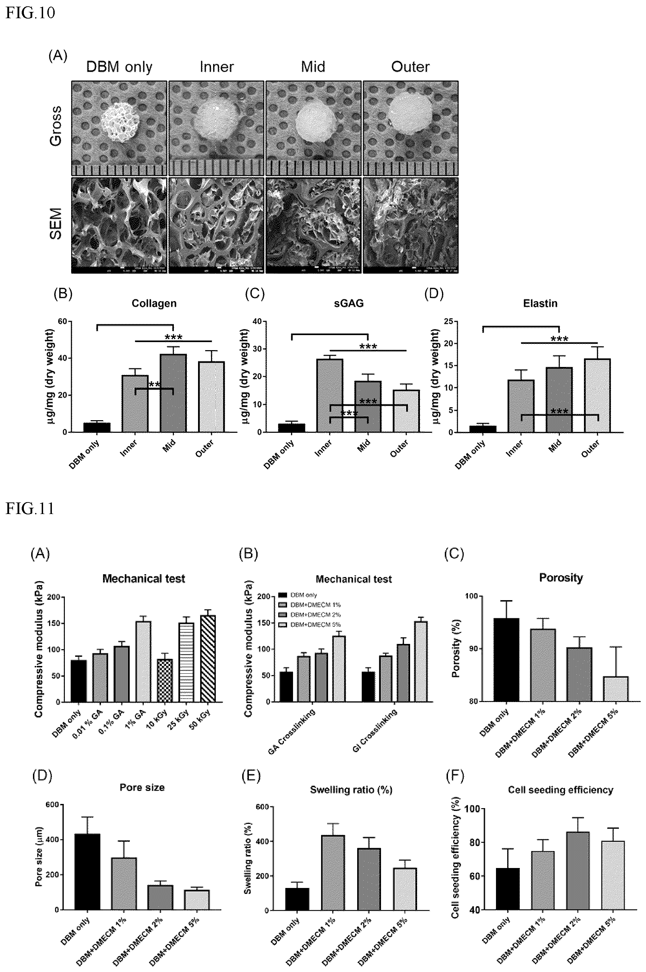

As a result of image analysis, as shown in FIG. 10(A), it was observed that the zone-specific DMECM-bioink is loaded in the porous structure of the DBM scaffold effectively by the processing technique.

In order to analyze the difference in the component content of the DBM scaffold loaded with the zone-specific DMECM-bioink, the content of collagen, sulfated glycosaminoglycans, and elastin was analyzed. Collagen was measured using S1000 (Biocolor, UK), sGAG using B1000 (Biocolor, UK), and elastin using Fastin assay (Biocolor, UK).

As a result, as shown in FIG. 10(B), it was determined that collagen showed the highest specific gravity when loaded with the Mid DMECM-bioink, sGAG had the highest specific gravity when loaded with the Inner DMECM-bioink, and elastin had the highest specific gravity when loaded with the Outer DMECM-bioink, indicating that the biochemical composition of the finally fabricated DBM scaffold is controllable depending on the selected type of the zone-specific DMECM-bioink.

### <Example 12> Physicochemical properties according to the cross-linking conditions of the DBM scaffold loaded with the zone-specific thermo-sensitive DMECM bioink

The DBM scaffold printed with the zone-specific DMECM-bioink manufactured according to one embodiment of the present disclosure may enhance the physicochemical properties through additional physical or chemical cross-linking methods. As a typical physical cross-linking method, there may be irradiation of ionization energy such as gamma rays or electron rays, but is not limited thereto. Chemical cross-linking methods that are used may primarily include glutaraldehyde or EDC/NHS, but are not limited thereto.

In the present embodiment, as a representative example of the chemical cross-linking method, the DMECM-DBM scaffold manufactured above was additionally cross-linked using glutaraldehyde. The cross-linking method was performed by adding the DMECM-DBM scaffold to 0.01 to 1% (w/v) glutaraldehyde solution and then stirring at 100 rpm for about 1 hour at room temperature. The chemically cross-linked DMECM-DBM scaffold was washed with PBS solution 3 times for 30 minutes repeatedly, followed by repeated washing 3 times with third distilled water.

On the other hand, as a representative example of the physical cross-linking method, the DMECM-DBM scaffold prepared above was additionally cross-linked using the gamma ray irradiation method. For the cross-linking method, the DMECM-DBM scaffold in the wet state with the printing completed was placed in an EP tube, wrapped in silver foil, and irradiated with gamma rays at an intensity of 10, 25, and 50 kGy, respectively. The compressive modulus was analyzed by a universal property tester to analyze the changes in the physicochemical properties of the DMECM-DBM scaffold following the additional cross-linking process. As a result of analysis, as shown in FIG. 11(A), it was found that the compressive modulus increased significantly with the increase in the concentration of the cross-linking agent and a dose of gamma irradiation.

Next, changes in the mechanical properties of the DBM-scaffold were observed according to the concentration of the printed DMECM-bioink. Referring to FIG. 11(B), it may be seen that the compressive modulus of the DBM scaffold increases significantly as the concentration of DMECM-bioink increases in both glutaraldehyde and gamma irradiation cross-linking methods.

Subsequently, as a result of analyzing the changes in the porosity and pore size of the DBM scaffold according to the concentration of the DMECM-bioink, it may be observed, as shown in FIGS. 11(C) and (D), that the porosity and pore size decrease as the concentration of DMECM-bioink increases. Therefore, it was determined that the manufacturing technology is adjustable to manufacture the DBM scaffold depending on the intended mechanical properties and porosity.

Specifically, when bioinks prepared at 1, 2, and 5% (w/v) were injected into the demineralized bone scaffold, the porosity was 93.7 ± 2.07%, 90.1 ± 2.1%, and 84.6 ± 5.7%, respectively, and the average diameter of the pores was shown to be about 295.1 ± 97.7 µm, about 137.9 ± 27.6 µm, and about 111.2 ± 18.5 µm, respectively.

It was determined that the porosity and pore size affect the swelling ratio of the scaffold and the cell seeding ratio (FIGS. 11E and F).

Specifically, when the bioink prepared at 1, 2, and 5% (w/v) were injected into the demineralized bone scaffold, the swelling ratio was 437.17 ± 65.8, 362.4 ± 59.7, and 247.5 ± 44.1, respectively, and the cell seeding rate of the scaffolds was shown to be about 74.9 ± 6.6, 86.3 ± 8.3, and 80.9 ± 7.4, respectively.

### <Example 13> Biomechanical properties of the DBM scaffold in accordance with a compact bone content

For the DBM scaffold according to one embodiment of the present disclosure, the spongy bone or the spongy bone in which a compact bone is included may be used. The spongy bone is observed in the center of bone or flat bone, has relatively high porosity and wide pore size compared to compact bone, and ensures excellent elasticity and resilience, making it easy to process and shape as a bioscaffold. The compact bones, on the other hand, are observed on the surrounding surface of the spongy bones and are denser than the spongy bones, making them mechanically stronger. The DMECM-DBM scaffold manufactured above may be applied to both the spongy bone and the DBM including spongy bone + compact bone.

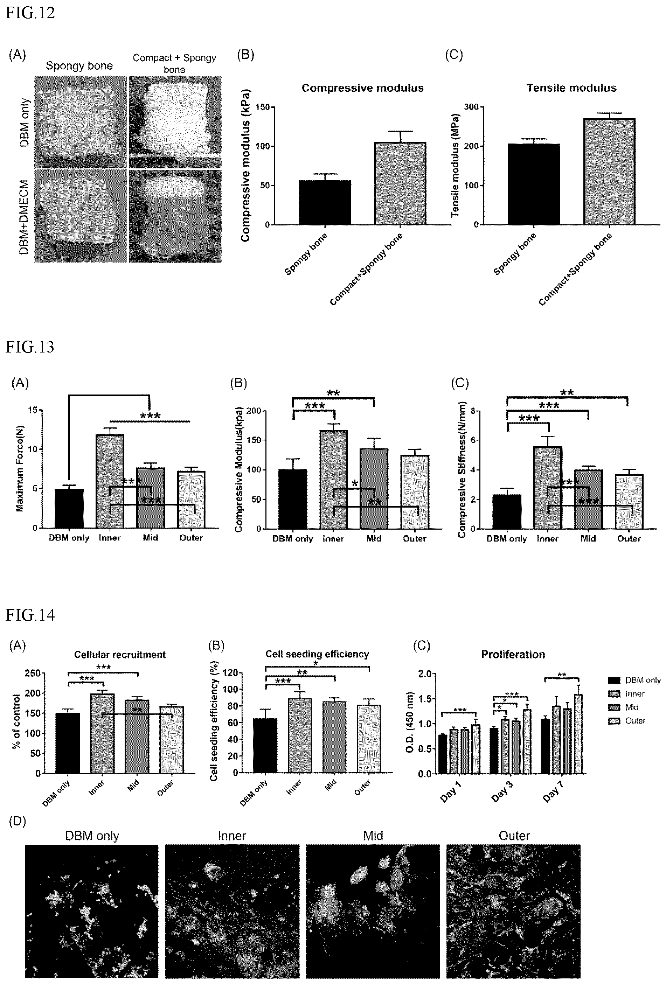

Referring to FIG. 12, it is possible to identify the difference in the physical properties depending on the structure of the DBM scaffold. DBM scaffolds containing compact bones and spongy bones in a ratio of 1:3 significantly increased the compressive modulus and tensile modulus compared to the DBM scaffolds containing only spongy bones (FIGS. 12B and 12C).

Therefore, it may be determined that the manufacturing technology according to the present disclosure is controllable for the manufacturing of the DMECM-DBM scaffold by selectively using the presence of a spongy bone or compact bone layer of DBM depending on the intended mechanical properties and porosity.

### <Example 14> Biomechanical properties of the DBM scaffold loaded with the zone-specific thermo-sensitive DMECM bioink

The physical strength of the DMECM-DBM scaffold according to one embodiment of the present disclosure is adjustable according to the type of the zone-specific DMECM-bioink.

Referring to FIG. 13, all DMECM-bioink treated groups showed increased compressive modulus compared to DBM only scaffolds, and in particular, DBM scaffolds loaded with the Inner DMECM-bioink showed the best compressive modulus of all groups.

Therefore, it may be found that the manufacturing technology according to the present disclosure may enable adjustment in the manufacturing of the DMECM-DBM scaffold by selectively using the DMECM-bioink depending on the intended mechanical properties.

### <Example 15> Cell affinity of the DBM scaffold loaded with the zone-specific thermo-sensitive DMECM bioink

To assess the difference in the impact of the zone-specific DMECM-DBM scaffold on cell behaviors, analysis was conducted on the cell proliferation and viable migration.

### 15-1. Analysis of fibro-cartilage cell migration ability by the zone-specific DMECM-DBM scaffold

A Boyden chamber assay was performed to determine whether the zone-specific DMECM-DBM scaffold has biochemical chemotaxis and is capable of promoting migration of meniscus-derived cells (meniscal cells).

As a result, as shown in FIG. 14(A), it may be seen that all DMECM-DBM scaffolds significantly promote migration of the meniscal cells compared to the control group (cell culture: 100%). In particular, the Inner DMECM-DBM scaffold showed the most significant chemotaxis for the meniscal cells of all groups, followed by the Mid and Outer DMECM-DBM scaffolds.

### 15-2. Analysis of fibro-cartilage cell seeding rate of the zone-specific DMECM-DBM scaffold

To assess the cell adhesion efficiency of the zone-specific DMECM-DBM scaffold, the cell seeding rate was analyzed.

After seeding 1×10⁶ cells into the DMECM-DBM scaffold, the cells were cultured on the culture dish at 37°C for 2 hours, and the number of cells that failed to adhere into the scaffold and escaped out of the culture dish was counted to calculate the number of cells adhered to the scaffold among the total cells.

As a result, as shown in FIG. 14(B), it was found that all the DMECM-DBM scaffold treated groups significantly promoted cell adhesion compared to the control group (DBM only scaffold), and in particular, the Inner DMECM-DBM scaffold group showed the highest cell seeding rate.

### 15-3. Analysis of fibro-cartilage cell proliferation ability of the zone-specific thermo-sensitive DMECM bioink

In order to determine the cell proliferation ability of the zone-specific DMECM-DBM scaffolds for meniscal cells, 1×10⁶ of meniscal cells per scaffold were seeded and cultured with cell culture at 37°C, followed by analysis of cell proliferation on days 1, 3, and 7 via WST assay.

As a result, as shown in FIG. 14(C), it was found that cell proliferation increased in the DMECM-DBM scaffold group of all groups compared to the DBM only group. In particular, it was observed that the Outer DMECM-DBM scaffold group increased cell proliferation most significantly among all groups.

Moreover, to analyze the long-term cell affinity of the zone-specific DMECM-DBM scaffold on meniscal cells, LIVE & DEAD assay was performed 4 weeks after cell culture.

As a result, it was found that the cells survived (Green) inside the DBM scaffold pores without the death (Red) of meniscal cells as shown in FIG. 14(D).

### <Example 16> In vitro fibrocartilage cell differentiation inducing properties of the DBM scaffold loaded with the zone-specific thermo-sensitive DMECM bioink

RT-PCR assay was performed to assess the effect on differentiation of human synovial membrane-derived mesenchymal stem cells (hMSCs) following culture in the zone-specific DMECM-DBM scaffolds.

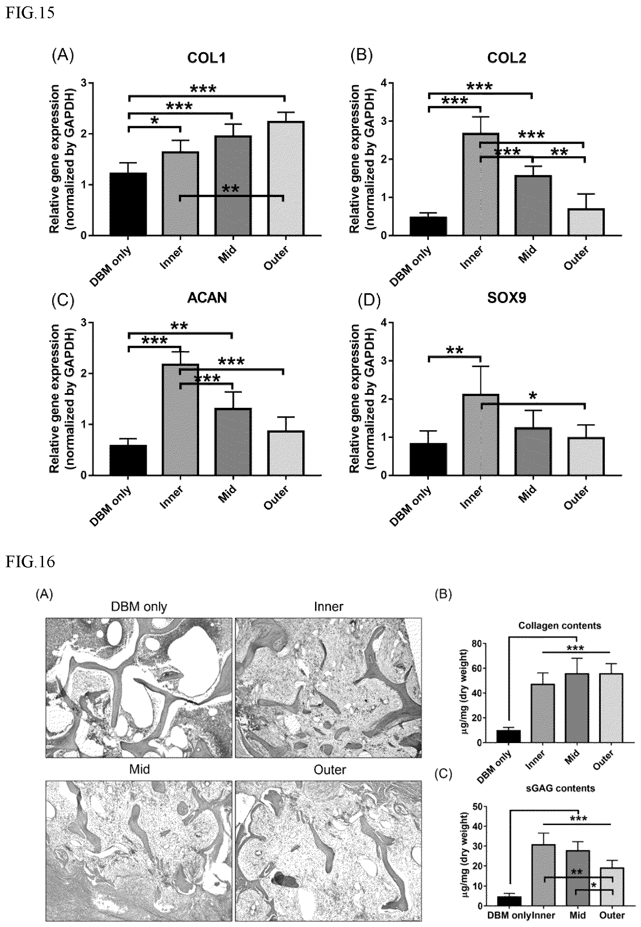

As a result, as shown in FIG. 15, it was observed that the zone-specific DMECM-DBM scaffolds in all groups significantly increased expression of cartilage and fibrous differentiation marker genes compared to the DBM only scaffold group. In particular, it was found that the Inner DMECM-DBM scaffold significantly enhanced the gene expression of type 2 collagen (COL2), aggrecan (ACAN), and SOX9, which are markers of cartilage differentiation.

On the other hand, in the case of type 1 collagen (COL1), a marker of fibrous differentiation, the highest gene expression was observed in the Outer DMECM-DBM scaffold. In the case of the Mid DMECM-DBM scaffold, the expression levels of fibrocartilage differentiation marker genes were observed to be medium to those of the Inner and Outer DMECM-DBM scaffolds.

### <Example 17> Assessment of in vivo fibrocartilage differentiation ability of the DBM scaffold loaded with the zone-specific thermo-sensitive DMECM bioink

### 17-1. Histological assessment of the zone-specific DMECM-DBM scaffold subcutaneously grafted in nude mice

In order to assess the in vivo artificial tissue formation capacity, hMSCs were seeded into the zone-specific DMECM-DBM scaffold and injected subcutaneously into nude mice (1×10⁶/scaffold). Four weeks after subcutaneous graft, histological assessment through Alcian blue staining was performed to assess the degree of tissue formation and differentiation by sacrificing the nude mice.

As a result, as shown in FIG. 16(A), it was found that the homogeneous cytoplasm was formed inside the porous structure of the DBM scaffold, and the cell distribution was also uniform. In particular, the strongest expression of sGAG was observed in the Inner DMECM-DBM scaffold group, determining that differentiation into cartilage tissues took place.

### 17-2. Identification of content of components in the zone-specific DMECM-DBM scaffold subcutaneously grafted in the nude mice

To identify the content of components in the artificial tissue formed in vivo following the grafting of hMSC and the zone-specific DMECM-DBM scaffolds, analysis was conducted on collagen and sGAG.

As a result, as shown in FIGS. 16(B) and (C), all DMECM-DBM scaffold groups significantly improved contents of collagen and sGAG compared to DBM only scaffolds. The collagen content was higher in the Mid and Outer DMECM-DBM scaffold groups compared to the Inner group (DBM only: 9.5 ± 2.7, Inner: 46.9 ± 9.3, Mid: 55.5 ± 12.5, Outer: 55.4 ± 8.3 µg/mg).

In the case of sGAG, the inner group showed the highest sGAG content, followed by the Mid and Outer groups (DBM only: 4.5 ± 1.8, Inner: 30.6 ± 5.8, Mid: 27.6 ± 4.6, Outer: 18.9 ± 3.9 µg/mg).

Therefore, it was determined that the Inner DMECM-DBM scaffold showed the characteristics of cartilaginous tissue differentiation, the Outer DMECM-DBM scaffold showed the characteristics of fibrous tissue differentiation, and the Mid DMECM-DBM scaffold showed the intermediate differentiation ability of the fibro-cartilage of the two scaffolds above.

### <Example 18> Preparation of an artificial meniscus scaffold using technology for manufacturing the DBM scaffold loaded with the zone-specific thermo-sensitive DMECM bioink

The technology to print the thermo-sensitive DMECM-bioink into the porous DBM scaffold enables mimicking of the zone-specificity of the meniscus. In the present disclosure, three different fluorescent dyes were combined to the Inner (Blue), Mid (Red), and Outer (Green) DMECM-bioink, respectively, which were then 3D printed on a DBM scaffold made in the form of meniscus, followed by fluorescence imaging and analysis on the internal microstructure.

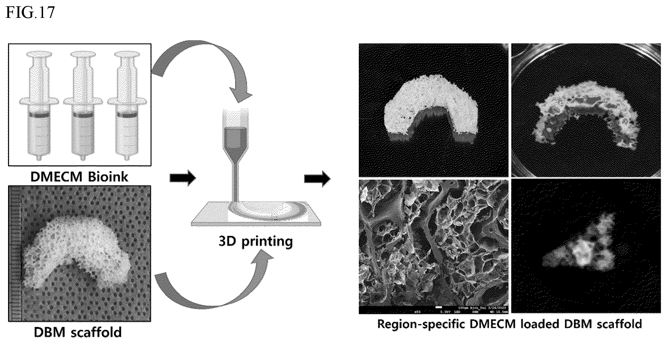

As a result, as shown in FIG. 17, it was found that the zone-specific DMECM-bioink is well loaded inside the DBM scaffold in the form of meniscus.

Overall, it is possible to adjust the physicochemical properties of the DBM scaffold loaded with the zone-specific meniscus-derived bioink according to the structure of the DBM scaffold as well as the type, concentration, and additional cross-linking process of the DMECM-bioink. It was identified that the DMECM-DBM scaffold may function as a chemoattractant that attracts cells, promotes cell adhesion and proliferation, and also induce differentiation into fibro-cartilage tissues in a zone-specific manner. It was determined that above technology may enable mimicking of the zone-specific biochemical properties shown in the meniscal tissues as well as the specificity of fibro-cartilage differentiation properties, with possibility to be applied for the reconstruction of various fibro-cartilage tissues including the meniscus.

While a specific part of the present disclosure has been described in detail above, it is clear for those skilled in the art that this specific description is merely preferred example embodiments, and the scope of the present disclosure is not limited thereby. In other words, the substantial scope of the present disclosure is defined by the appended claims and their equivalents.

## Claims

1. A bioink composition for 3D printing comprising an extracellular matrix derived from fibrocartilage composite tissue,
wherein the fibrocartilage composite tissue is at least one selected from tissues compartmentalized into inner, mid, and outer meniscal tissues depending on anatomical positions, where the inner has cartilaginous properties, the outer has fibrous properties, and the mid has the property that the cartilage and fibrous properties are converted, such that physicochemical properties vary depending on the compartmentalized tissues.

2. The bioink composition of claim 1, wherein the composition promotes migration, adhesion, and proliferation of fibrocartilage cells.

3. The bioink composition of claim 1, wherein the composition differentiates into fibrocartilage composite tissue in vitro or in vivo to induce formation of artificial tissues.

4. The bioink composition of claim 1, wherein the bioink composition comprising the extracellular matrix derived from the tissue compartmentalized into the outer induces angiogenesis.

5. The bioink composition of claim 1, wherein the bioink composition comprising the extracellular matrix derived from the tissue compartmentalized into the inner or the mid inhibits angiogenesis.

6. The bioink composition of claim 1, wherein the composition exists in a liquid form at room temperature and undergoes gelation at temperatures of 37°C or higher.

7. A bone graft composition comprising a demineralized bone matrix scaffold, wherein the bioink composition according to any one of claims 1 to 6 is included in the scaffold and cross-linked.

8. The bone graft composition of claim 7, wherein the bioink composition has a concentration of 0.1 to 10 weight-volume (w/v) percent.

9. The bone graft composition of claim 7, wherein the demineralized bone matrix scaffold comprises spongy bone, compact bone, or a combination thereof.

10. A method of manufacturing a bioink for 3D printing, the method comprising:
obtaining and pulverizing fibrocartilage composite tissue;
decellularizing the pulverized tissue to obtain and pulverize an extracellular matrix; and
water-solubilizing the pulverized extracellular matrix by enzymatic treatment,
wherein the fibrocartilage composite tissue is one or more selected from tissues compartmentalized into inner, mid, and outer meniscal tissues depending on anatomical positions, where the inner has cartilaginous properties, the outer has fibrous properties, and the mid has the property that the cartilage and fibrous properties are converted, such that physicochemical properties vary depending on the compartmentalized tissues.

11. The method of claim 10, wherein the water-solubilizing of the pulverized extracellular matrix by enzymatic treatment is performed by enzymatically treating the extracellular matrix with pepsin in an acidic solution.

12. A method of manufacturing a bone graft, the method comprising:
preparing a demineralized bone matrix scaffold;
preparing a bioink comprising an extracellular matrix derived from fibrocartilage composite tissue according to the method of claim 10 or 11; and
injecting the prepared bioink into the prepared demineralized bone matrix scaffold to carry out cross-linking.

13. The method of claim 12, wherein the preparing of the demineralized bone matrix scaffold comprises:
obtaining osteo-cartilage composite tissue and removing minerals from the composite tissue; and
removing cartilage tissues from the composite tissue from which the minerals are removed.

14. The method of claim 12, wherein the injecting of the prepared bioink into the prepared demineralized bone matrix scaffold to carry out cross-linking is performed by 3D printing the bioink and then injecting into a porous structure in the demineralized bone matrix scaffold to carry out cross-linking at a temperature of 37°C or higher.

15. The method of claim 12, further comprising, after injecting of the prepared bioink into the prepared demineralized bone matrix scaffold to carry out cross-linking, carrying out additional cross-linking using one or more physical or chemical methods.

16. The method of claim 15, wherein the physical method is a method of irradiating ionization energy selected from gamma rays or electron rays.

17. The method of claim 15, wherein the chemical method is a method of using a cross-linking agent selected from glutaraldehyde or 1-ethyl-3-(3-dimethylaminopropyl)carbodiimide/N-hydroxysuccinimide (EDC/NHS).

18. A method of manufacturing an artificial tissue, comprising 3D printing the bioink composition according to any one of claims 1 to 6.

19. The method of claim 18, wherein the 3D printing is performed, using a nozzle with an average diameter of 300 to 600 µm, at a rate of 1 to 2 mm/sec under air pressures of less than 80 kPa at a temperature of 37°C or higher.

20. An artificial tissue which is manufactured by the method according to claim 18 and has characteristics of fibrocartilage composite tissue.
